# EUROPEAN PATENT SPECIFICATION

(11) **EP 2 292 752 B1**
(45) Date of publication and mention of the grant of the patent: **23.09.2015**
(21) Application number: 09750532.5
(22) Date of filing: 18.05.2009
(51) Int. Cl.: C12N 11/04, C12N 11/08, C12N 11/10, C12P 7/06, C12P 7/56, A61K 9/48

(54) **SEAMLESS CAPSULE**
NAHTLOSE KAPSEL
CAPSULE SANS SOUDURE

(30) Priority: 19.05.2008 JP 2008131114
(43) Date of publication of application: 09.03.2011
(73) Proprietor: Morishita Jintan Co., Ltd., Osaka-shi Osaka 540-8566 (JP)
(72) Inventor: YOSHIKADO, Masatomo, Osaka-shi Osaka 540-8566 (JP); NAKATSUJI, Masaaki, Osaka-shi Osaka 540-8566 (JP); ASADA, Masanori, Osaka-shi Osaka 540-8566 (JP); KAMAGUCHI, Ryosei, Osaka-shi Osaka 540-8566 (JP); TAKADERA, Takahide, Hiratsuka-shi Kanagawa 254-8562 (JP); MURAMATSU, Toshimitsu, Hiratsuka-shi Kanagawa 254-8562 (JP)
(74) Representative: Koepe, Gerd L.
(86) International application number: PCT/JP2009/059123
(87) International publication number: WO 2009/142170

(56) References cited:
- EP-A1- 1 407 678
- JP-A- S 562 908
- JP-A- H08 103 271
- JP-A- H11 302 158
- JP-A- H11 508 477
- JP-A- S61 151 127
- JP-A- S62 201 823
- JP-A- 2000 086 525
- JP-A- 2001 245 660
- JP-A- 2003 088 747
- JP-A- 2003 325 638
- JP-A- 2006 061 097
- JP-A- 2006 501 281
- MASANORI ASADA: 'Funtai Gijutsu·Capsule-ka Gijutsu, Seamless Capsule-ka Gijutsu no Tokucho to sono Oyo' THE FOOD INDUSTRY vol. 47, no. 24, 30 November 2004, pages 45 - 51, XP001077078
- RYOSEI KAMAGUCHI: 'Yuyosei Kin no Capsule-ka Gijutsu' CHEMICAL ENGINEERING OF JAPAN vol. 71, no. 2, 2007, pages 123 - 126, XP008146610
- RYOSEI KAMAGUCHI: 'Taso Seamless Capsule no Shokuhin eno Oyo' FOOD STYLE 21 vol. 13, no. 3, 01 March 2009, pages 74 - 75, XP008161813
- 'Dai 60 Kai Abstracts of the Annual Meeting of the Society for Biotechnology, Japan', 11 July 2008 article MASANORI ASADA ET AL.: 'Seikin Iri Seamless Capsule no Kenkyu Kaihatsu', page 222, XP008146611

## Description

### Technical Field

The present invention relates to a seamless capsule that contains a biocatalyst such as an enzyme or a living cell and that is applicable to a bioreactor and the like.

### Background Art

Since 1950, it has been increasing that in order to reduce environmental burdens, a biocatalyst, such as an organism-derived enzyme, a microorganism, or an animal- or plant-derived cell or tissue, is placed into a reaction vessel to produce substance, as it is called, production of substance by a bioreactor. Techniques in the bioengineering field and the bioscience field are applied to the bioreactor. For example, for effective use of a biocatalyst, a technique for immobilizing the biocatalyst is utilized in a bioreactor.

For example, methods including carrier binding, cross-linking, entrapment, and the combinations thereof can be used for the immobilization of biocatalyst. In particular, entrapment (specifically, entrapment using a macromolecular gel such as agar, carrageenan, alginate, photocurable resin, or polyacrylamide) is used for the immobilization of microorganisms, or cells or tissue from animals or plants.

In detail, conventionally, microorganisms and living cells are suspended in a sol of macromolecule in which a gel has not been formed, and the sol is then gelled for entrapment. Therefore, living cells near the surface of the gel are only effectively used, and the reactivity per living cell is poor. Moreover, some living cells may fall out of the gel matrix.

Various methods for entrapment have been proposed to address these problems (for example, European Patent Application EP 1 407 678 A1 and Patent Documents 1 to 3).

European Patent Application EP 1407 678 A1 discloses capsules which contain liquid in which vital cells or tissues are suspended and in which these cells or tissues can be grown.

Patent Document 1 discloses a hollow microcapsule of a porous outer shell and encapsulation of microorganisms or the like in the microcapsule. Specifically, it is disclosed that an organic phase, which contains calcium alginate beads in which yeast is entrapped, is dispersed in an aqueous phase to prepare an O/W emulsion, and this emulsion is dried to give a microcapsule (coacervation method), and calcium alginate included in the microcapsule is dissolved and removed by washing with hydrochloric acid to enclose yeast in the hollow microcapsule. However, for this microcapsule, since polystyrene is mainly used in the shell of capsule, a harmful organic solvent may be used during formation of capsule. The microcapsules obtained by this coacervation method are not uniform in capsule diameter and thickness of shell, and therefore the reactivity of yeasts in the core of the capsule may be uncontrollable.

Patent Document 2 discloses a seamless capsule in which living cells and tissue are encapsulated. Patent Document 3 discloses a three-layer seamless capsule having a shell of photocurable resin. For the seamless capsules as disclosed in these documents, since water is in the core and the fluid containing living cells to be enclosed is an aqueous fluid, care must be taken to avoid contamination. Also, prolonged time for drying, complicated process, increased cost may be caused for production of the seamless capsules.

Patent Document 4 discloses a method for producing an immobilized microorganism, by preparing a suspension containing a prepolymer having a molecular weight of 3500 to 20000, a cross-linking agent having a molecular weight of 71 or greater and having a ratio of its molecular weight to that of the prepolymer is 0.045 or less, and a microorganism in specific proportions, and polymerizing the suspension to produce the included and immobilized microorganism in the cross-linked polymer. For the included and immobilized microorganism (beads) obtained by this method, it is problematic that the increased microorganisms on the surface with the progression of the reaction transfer into the reaction solution to cause the cloud.

Completely away from the entrapment, Patent Document 5 discloses a stable live bacterial preparation characterized in that a dry powder containing a live bacterium having a control action on the intestinal function is suspended in a fat or oil, for the storage stability of the live bacterium, and it also discloses that the live bacterial preparation is filled in a soft capsule for use. Patent Document 6 discloses a two-layer capsule in which an oil containing dispersed beneficial enterobacterium is filled in a capsule having a diameter of 3 mm or less, for delivering the beneficial enterobacterium via oral ingestion to the intestine.

Other than those described above, there is demand for capsules applicable to a bioreactor in which a biocatalyst is immobilized.

### Prior Art Documents

### Patent Documents

Patent Document 1: Japanese Laid-Open Patent Publication No. 2003-88747
Patent Document 2: Japanese Laid-Open Patent Publication No. 2001-245660
Patent Document 3: Japanese Laid-Open Patent Publication No. 2003-325638
Patent Document 4: Japanese Laid-Open Patent Publication No. 2006-61097
Patent Document 5: Japanese Laid-Open Patent Publication No. S56-2908
Patent Document 6: Japanese Laid-Open Patent Publication No. S62-201823

### Summary of the Invention

### Problems To Be Solved By_the Invention

It is an object of the present invention to provide a seamless capsule applicable to a bioreactor.

### Means for Solving the problems

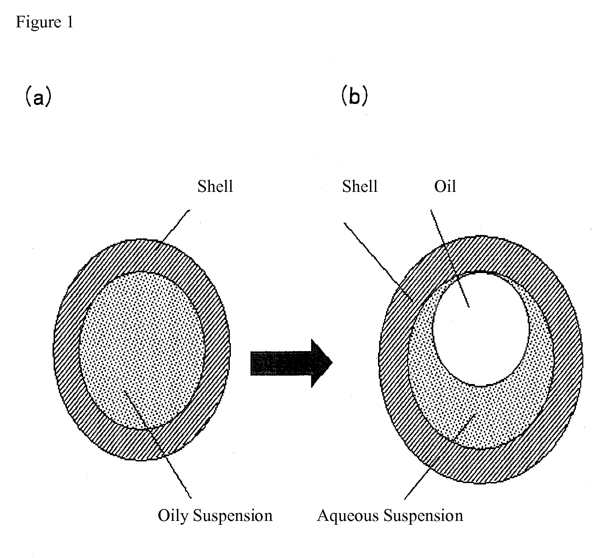

The inventors have conducted extensive research to solve the problems described above, and found that a seamless capsule having a pseudo three-layer structure of outer layer/oil layer/aqueous layer (see Fig. 1(b)) is applicable to a bioreactor. The capsule is obtained by immersing, in an aqueous fluid, a two-layered seamless capsule (see Fig. 1 (a)) composed of an inner layer of a suspension composition that contains a biocatalyst suspended in an oily substance and an outer layer of a shell composition that contains a water permeable substance, and contains an aqueous fluid enclosed therein. That is, since the two-layered seamless capsule has an outer layer of water permeability, for example, by immersing the seamless capsule having a microorganism (biocatalyst) encapsulated in an aqueous fluid such as a liquid medium, the liquid medium can be incorporated into the capsule through the outer layer while retaining the microorganism in the capsule, leading to a seamless capsule having a pseudo three-layer structure of outer layer/oil layer/aqueous layer as shown in Fig. 1(b). In the seamless capsule having the pseudo three-layer structure, the microorganism can utilize components of the medium to produce a metabolite (useful substance) and the produced metabolite (useful substance) can be released from the inside of the capsule to the outside of the capsule.

According to the present invention, a seamless capsule having a pseudo three-layer structure of outer layer/oil layer/aqueous layer, obtained by immersing in an aqueous fluid a two-layered seamless capsule comprising an inner layer comprising a suspension composition comprising a biocatalyst suspended in an oily substance and an outer layer comprising a shell composition comprising a water permeable substance.

In one embodiment, the biocatalyst is at least one selected from the group consisting of enzymes, microorganisms, animal cells, plant cells, and plant tissues.

In one embodiment, the biocatalyst is at least one selected from the group consisting of microorganisms, animal cells, plant cells, and plant tissues, and the biocatalyst is activated by the

The invention relates to a seamless capsule that can be stably stored for a long period of time in the form of a two-layered seamless capsule, comprising an inner layer comprising a suspension composition, and an outer layer comprising a shell composition and having water permeability, wherein the suspension composition of the inner layer comprises a biocatalyst suspended in an oily substance, and the shell composition of the outer layer comprises a water permeable substance, wherein the two-layered seamless capsule is forming a pseudo three-layer structure of outer layer/oil layer/aqueous layer, when being immersed in an aqueous fluid; wherein the biocatalyst is at least one selected from the group consisting of microorganisms, animal cells, plant cells, and plant tissues.

The invention also relates to a bioreactor comprising the above seamless capsule.

Furthermore, the invention relates to a method for producing the seamless capsule according to any one of claims 1 to 4, comprising immersing in an aqueous fluid a two-layered seamless capsule, comprising an inner layer comprising a suspension composition, and an outer layer comprising a shell composition and having water permeability, wherein the suspension composition of the inner layer comprises a biocatalyst suspended in an oily substance and the shell composition of the outer layer comprises a water permeable substance.

Finally, the invention also relates to the use of the above seamless capsule as a bioreactor. pseudo three-layer structure of outer layer/oil layer/aqueous layer.

In one embodiment, the shell composition comprises at least one selected from the group consisting of carrageenan, agar, glucomannan, alginate, acrylate-based oligomers, unsaturated polyester-based oligomers, epoxy-based oligomers, vinyl ether-based oligomers, polyene-thiol-based oligomers, and cinnamate-based oligomers.

In one embodiment, the oily substance is at least one selected from the group consisting of olive oil, jojoba oil, corn oil, rapeseed oil, lard, beef tallow, whale oil, castor oil, soybean oil, rice oil, rice germ oil, coconut oil, palm oil, cacao seed oil, avocado oil, macadamia nut oil, squalane, mink oil, turtle oil, hydrocarbons having 8 to 30 carbon atoms, beeswax, carnauba wax, rice wax, lanolin, liquid paraffin, petrolatum, fatty acids having 4 to 30 carbon atoms, esters of fatty acids having 4 to 30 carbon atoms and sucrose, esters of fatty acids having 4 to 30 carbon atoms and glycerol, fatty alcohols having 4 to 30 carbon atoms, and esters of fatty acids having 4 to 30 carbon atoms and fatty alcohols having 4 to 30 carbon atoms.

The present invention provides a bioreactor comprising the seamless capsule having a pseudo three-layer structure of outer layer/oil layer/aqueous layer.

The present invention provides a method for producing a seamless capsule having a pseudo three-layer structure of outer layer/oil layer/aqueous layer, comprising:
immersing in an aqueous fluid a two-layered seamless capsule comprising an inner layer comprising a suspension composition comprising a biocatalyst suspended in an oily substance and an outer layer comprising a shell composition comprising a water permeable substance.

In one embodiment, the two-layered seamless capsule is obtained
using a capsule production device equipped with a concentric double nozzle having, from the innermost, a first nozzle and a second nozzle,
by a method comprising:
simultaneously extruding the suspension composition comprising a biocatalyst suspended in an oily substance from the first nozzle and the shell composition comprising a water permeable substance from the second nozzle into a fluid.

### Effects of the Invention

The seamless capsule of the invention is obtained by immersing in an aqueous fluid a two-layered seamless capsule composed of an inner layer of a suspension composition that contains a biocatalyst suspended in an oily substance and an outer layer of a shell composition that contains a water permeable substance. In the two-layered seamless capsule, the biocatalyst is suspended in the oily substance in the inner layer, and is suitably isolated from water (that is, it is in a dry state). Therefore, the biocatalyst is seldom contaminated and is stable for a long period of time. Also, the outer layer that allows an aqueous fluid to penetrate allows only an aqueous liquid to transfer between the inside and the outside of the capsule without allowing the biocatalyst to pass to the outside. Therefore, according to the invention, for example, by immersing the two-layered seamless capsule having a microorganism (biocatalyst) encapsulated in a liquid medium, the liquid medium is incorporated into the capsule while retaining the microorganism (biocatalyst) in the capsule, thereby giving a pseudo three-layer structure of outer layer/oil layer/aqueous layer, and then a metabolite (useful component) produced by the microorganism can be released from the inside to the outside of the capsule. Thus, the seamless capsule having a pseudo three-layer structure of outer layer/oil layer/aqueous layer of the invention is useful in that it is applicable to a bioreactor as a material for biocatalyst immobilization. Moreover, according to the invention, a two-layered seamless capsule is prepared and a liquid substrate (an aqueous fluid such as a liquid medium) is penetrated from the outer layer into the inner layer of the capsule as described above, and therefore it is not necessary to encapsulate the liquid substrate in advance. The seamless capsule of the invention can be more stably stored in the form of a two-layered seamless capsule in which a biocatalyst is encapsulated in a high level, until it is ready to be used, compared to conventional immobilized biocatalysts. Therefore, the seamless capsule of the invention is more efficient than conventional immobilized biocatalysts in which a biocatalyst is mixed in a gel. Furthermore, unlike conventional three-layered seamless capsules, the seamless capsule of the invention is not required to cool a biocatalyst-containing aqueous suspension of the innermost layer so as to prevent inactivation of the biocatalyst. In addition, although three-layered seamless capsules which might be obtained by conventional production methods have limitations to reduce the diameter, it is possible that the seamless capsule having a pseudo three-layer structure of outer layer/oil layer/aqueous layer of the invention is stable in a further reduced diameter because it is based on a two-layered seamless capsule.

### Brief Description of Drawings

Fig. 1 shows schematic cross-sectional illustrations of seamless capsules.
Fig. 2 is a schematic illustration showing an example of a capsule production device for producing the seamless capsule of the present invention.
Fig. 3 provides photographs showing the capsule state change during the process of producing the seamless capsule of the present invention.

### Mode for Carrying Out the Invention

The seamless capsule of the invention is obtained by immersing a two-layered seamless capsule having the specified inner layer and outer layer in an aqueous fluid. The seamless capsule of the invention has a pseudo three-layer structure of outer layer/oil layer/aqueous layer.

### Two-layered seamless capsule

A two-layered seamless capsule for use in the present invention is composed of an inner layer of a suspension composition that contains a biocatalyst suspended in an oily substance and an outer layer of a shell composition that contains a water permeable substance.

### (1) Inner layer (suspension composition)

The inner layer is composed of a suspension composition that contains a biocatalyst suspended in an oily substance. The biocatalyst is not particularly limited. The biocatalyst can activate the reaction in the presence of an aqueous fluid, i.e., in the pseudo three-layer structure of outer layer/oil layer/aqueous layer, and examples include those used as the reaction element in bioreactor and the like. Specifically, enzymes, microorganisms (such as lactic acid bacteria), animal cells (such as islets of Langerhans and adipose cells), plant cells (such as dedifferentiated calluses), plant tissues (such as embryoids, adventitious buds, multiple shoots, shoot tips, growing points, protocorm-like bodies, adventitious roots, and hairy roots), and the like may be used. Biocatalysts may be used alone or in a combination of two or more.

The biocatalyst, for example, in a powder form (such as enzyme or bacterial powder), is contained in the suspension composition in a proportion of 0.001 to 30 wt% and preferably 0.01 to 20 wt%. The biocatalyst, in a form of living cells, including microorganisms, animal cells, plant cells, plant tissues, and the like, is contained at about 1 cell/ml to 5×10¹¹ cells/ml and preferably about 1×10³ cells/ml to 1×10¹¹ cells/ml in the suspension composition.

The oily substance is not particularly limited as long as it is liquid during the production of the two-layered seamless capsule. It is preferable to use an oily substance that has a melting point of -30°C to 60°C to prevent the inactivation of biocatalyst. Examples of such oily substances include olive oil, jojoba oil, corn oil, rapeseed oil, lard, beef tallow, whale oil, castor oil, soybean oil, rice oil, rice germ oil, coconut oil, palm oil, cacao seed oil, avocado oil, macadamia nut oil, squalane, mink oil, turtle oil, hydrocarbons having 8 to 30 carbon atoms, beeswax, carnauba wax, rice wax, lanolin, liquid paraffin, petrolatum, fatty acids having 4 to 30 carbon atoms, esters of fatty acids having 4 to 30 carbon atoms and sucrose (sucrose fatty acid esters), esters of fatty acids having 4 to 30 carbon atoms and glycerol (fatty acid monoglycerides, fatty acid diglycerides, or fatty acid triglycerides), fatty alcohols having 4 to 30 carbon atoms, esters of fatty acid having 4 to 30 carbon atoms and fatty alcohols having 4 to 30 carbon atoms, and the like. The constituent fatty acids of the hydrocarbons, fatty acid glycerides, sucrose fatty acid esters, and fatty acids as mentioned above may be saturated fatty acids or unsaturated fatty acids. Also, the hydrocarbon groups constituting the fatty alcohols may be saturated hydrocarbon groups or unsaturated hydrocarbon groups. Oily substances may be used alone or in a combination of two or more. The viscosity or the density of the oily substance is not particularly limited and may be suitably adjusted.

In use of an ester of a fatty acid having 4 to 30 carbon atoms and glycerol (in particular, fatty acid triglyceride) or oil or fat containing such an ester as the oily substance, lipase may be added to the inner layer (suspension composition) to degrade the oil or fat after the preparation of the two-layered seamless capsule. By immersing the two-layered seamless capsule in water, the capsule swells so as to allow water to penetrate inside of the capsule. The lipase starts to act with permeated water, so that the oil or fat is hydrolyzed. The hydrolyzed product of the oil or fat is soluble in the permeated water and can be released from the inside to the outside of the capsule. Therefore, the volume of the oil or fat in the capsule is reduced, and correspondingly an aqueous fluid can be readily incorporated into the capsule, making the contact of the biocatalyst with the aqueous component (substrate) more efficiently.

The suspension composition is obtained by suspending the biocatalyst in the oily substance. The method for suspending may be any of methods commonly used by those skilled in the art, such as stirring.

### (2) Outer layer (outer-layer composition)

The outer layer is composed of a shell composition that contains a water permeable substance, and that may contain other components as necessary. Therefore, the outer layer allows an aqueous fluid to permeated therethrough and has so expandability that the obtained two-layered seamless capsule can be incorporate an aqueous liquid when it is immersed in the aqueous fluid. The water permeable substance is preferably at least one of gelling agents and photocurable resins, and a shell obtained therefrom exhibits a great permeability to an aqueous fluid. Although the swelling of the shell itself with water is not large, the shell has so expandability that the seamless capsule can readily incorporate the aqueous fluid thereinto. The aqueous fluid as used herein refers to water or an aqueous solution containing an aqueous component. The aqueous component refers to a water soluble material that can serve as a substrate of a biocatalyst and is suitably selected depending on the type of biocatalyst. Examples of aqueous components include components (including carbon sources, nitrogen sources, minerals, and the like) of medium.

### (2-1) Gelling agent

Examples of gelling agents include polysaccharides such as carrageenan, agar, glucomannan, alginate, gellan gum, xanthan gum, locust bean gum, pectin, psyllium seed gum, guar gum, furcellaran, arabinogalactan, arabinoxylan, gum arabic, dextrin, modified dextrin, starch, modified starch, pullulan, carboxymethylcellulose salts, and the like. Carrageenan, agar, glucomannan, and alginate are preferable. The gelling agent is contained in the shell composition in a solid content of preferably 0.1 to 40 wt% and more preferably 0.3 to 30 wt%.

### (2-2) Photocurable resin

A photocurable resin refers to a resin using a reaction caused by photoirradiation. Usually, a photopolymerizable monomer, a photopolymerizable oligomer, or an addition polymer of a photopolymerizable monomer or a photopolymerizable oligomer is used. Photocurable resins may be used alone or in a combination of two or more. It is preferable to use a photocurable resin in combination with a polymerization initiator.

Examples of the photopolymerizable oligomer include oligomers using radical polymerization and oligomers using cationic polymerization reaction. The number average molecular weight of the photopolymerizable oligomer is in the range of 300 to 30000 and preferably 500 to 20000.

The photopolymerizable oligomer using radical polymerization has functional group(s), such as a (meth)acriloyl group, a vinyl group, or the like, examples of which include urethane (meth)acrylate-based oligomers, epoxy (meth)acrylate-based oligomers, ester (meth)acrylate-based oligomers, (meth)acrylate-based oligomers, unsaturated polyester-based oligomers, polyene-thiol-based oligomers, and cinnamate-based oligomers. Specific examples include resins having a photopolymerizable ethylenic unsaturated group at each terminal of a polyalkylene glycol (such as polyethylene glycol di(meth)acrylate, polypropylene glycol di(meth) acrylate, polyalkylene glycol di(meth)acrylate in which the number of carbon atoms of the alkyl chain is 4 to 10, pentaerythritol tetra(meth)acrylate, and dipentaerythritol hexa(meth)acrylate), high acid value unsaturated polyesters, high acid value unsaturated polyepoxides, anionic unsaturated acrylic resins, cationic unsaturated acrylic resins, unsaturated polyamides, and the like. The term "(meth)acrylate" refers to at least one of acrylate and methacrylate. For example, the "polyethylene glycol di(meth)acrylate" refers to at least one of polyethylene glycol diacrylate and polyethylene glycol dimethacrylate.

The photopolymerizable oligomer using cationic polymerization reaction has functional group(s) such as an epoxy group, a vinyl ether group, or the like, examples of which include epoxy-based oligomers and vinyl ether-based oligomers.

In particular, among the photopolymerizable oligomers, acrylate-based oligomers, unsaturated polyester-based oligomers, polyene-thiol-based oligomers, cinnamate-based oligomers, epoxy-based oligomers, and vinyl ether-based oligomers are preferably used.

In particular, among the photocurable resins, resins containing hydrophilic ionic and/or nonionic group(s), such as a hydroxy group, an amino group, a carboxyl group, a phosphate group, a sulfonate group, or an ether bonding, enough for homogeneous dispersion in an aqueous medium, are suitably used. Such photocurable resins are disclosed in, for example, Japanese Patent Publication No. S55-40, Japanese Patent Publication No. S55-20676, and Japanese Patent Publication No. S62-19837. Specifically, hydrophilic resin compounds having at least two ethylenic unsaturated bonds within a molecule, high acid value unsaturated polyesters, high acid value unsaturated epoxides, anionic unsaturated acrylic resins, unsaturated polyamide, and the like are suitably used. Among these examples, hydrophilic resin compounds having at least two ethylenic unsaturated bonds within a molecule are preferably used.

The hydrophilic resin compounds having at least two ethylenic unsaturated bonds within a molecule include resins having a photopolymerizable ethylenic unsaturated group at each terminal of a polyalkylene glycol. For example, (1) polyethylene glycol di(meth)acrylates obtained by treating polyethylene glycol having a molecular weight of 400 to 6000 with 2 mol of (meth)acrylic acid to esterify the hydroxy groups at both the terminals of polyethylene glycol; (2) polypropylene glycol di(meth)acrylates obtained by treating polypropylene glycol having a molecular weight of 200 to 4000 with 2 mol of (meth)acrylic acid to esterify the hydroxy groups at both the terminals of polypropylene glycol; (3) urethane derivatives having unsaturated groups and polyethylene glycol obtained by treating 1 mol of polyethylene glycol having a molecular weight of 400 to 6000 with 2 mol of a diisocyanate compound (such as tolylene diisocyanate, xylylene diisocyanate, and isophorone diisocyanate), so that the hydroxy groups are converted to urethane groups at both the terminals of polyethylene glycol, and further adding thereto 2 mol of an unsaturated monohydroxy compound (such as 2-hydroxyethyl (meth)acrylate, 2-hydroxypropyl (meth)acrylate, 3-hydroxypropyl (meth)acrylate, 4-hydroxybutyl (meth)acrylate, trimethylolpropane di(meth)acrylate, and pentaerythritol tri(meth)acrylate); and (4) urethane derivatives having unsaturated groups and polypropylene glycol obtained by treating 1 mol of polypropylene glycol having a molecular weight of 200 to 4000 with 2 mol of a diisocyanate compound, so that the hydroxy groups are converted to urethane groups at both the terminals of polypropylene glycol, and further adding thereto 2 mol of an unsaturated monohydroxy compound.

Examples of the high acid value unsaturated polyester mentioned above include salts of unsaturated polyesters having an acid value of 40 to 200 obtained by the esterification of polycarboxylic acids having an unsaturated bond with polyalcohols, and the like.

Examples of the high acid value unsaturated epoxide mentioned above include unsaturated epoxy resins having an acid value of 40 to 200 and the like. Such a resin is, for example, that obtained by preparing an adduct of an epoxy resin with an unsaturated carboxylic compound (such as a (meth)acrylate) and adding an acid anhydride to the hydroxyl group remaining in the adduct.

Examples of the anionic unsaturated acrylic resin mentioned above include resins in which a photopolymerizable ethylenic unsaturated group is introduced into a copolymer derived from at least two (meth)acryl-based monomers of (meth)acrylic acid and (meth)acrylic esters and having a carboxyl group, a phosphate group, and/or a sulfonate group, and the like.

The unsaturated polyamide mentioned above is, for example, that obtained by adding adducts of a diisocyanate (such as tolylene diisocyanate and xylylene diisocyanate) and an ethylenic unsaturated hydroxy compound (such as 2-hydroxyethyl acrylate) to a water soluble polyamide such as gelatin.

Among such photocurable resins, resins having a polymerizable ethylenic unsaturated group at each terminal of polyalkylene glycol can be particularly advantageously used in the present invention, and typical examples are commercially available from Kansai Paint Co., Ltd., under the trade names of ENT-1000, ENT-2000, ENT-3400, ENT-4000, ENTG-2000, ENTG-3800, and the like.

The photocurable resin is contained in the shell composition in a solid content of preferably 10 to 99 wt%, more preferably 20 to 90 wt%, and still more preferably 40 to 90 wt%.

### (2-3) Other components

Examples of other components that may be contained in the shell composition include gelling aids, water soluble compounds having an unsaturated bond, polymerization initiators, photosensitizers, coloring agents, and pore-forming agents. In use of a gelling agent, it is preferable to use a gelling aid. In use of a photocurable resin, it is preferable to use a polymerization initiator, especially a photopolymerization initiator.

A gelling aid is used to enhance the strength of a shell that contains a gelling agent. The gelling aid may be suitably selected depending on the type of gelling agent. Examples of gelling aids include water soluble polyalcohols such as sorbitol, mannitol, glycerol, propylene glycol, polyethylene glycol, glucose, fructose, galactose, arabinose, mannose, rhamnose, maltose, raffinose, sucrose, erythritol, maltitol, trehalose, lactose, xylose, and like; alkali metal salts, alkaline earth metal salts, and ammonium salts. Gelling aids may be used alone or in a combination of two or more. In particular, where alginate, gellan gum, pectin, or carrageenan is contained as a gelling agent, it is preferable to concomitantly use an alkali metal salt, an alkaline earth metal salt, an ammonium salt, or the like as a gelling aid. The gelling aid is contained in the shell composition in a solid content of preferably 0.1 to 30 wt% and preferably 0.5 to 20 wt%.

A water soluble compound having an unsaturated bond is used to enhance the strength of a shell that contains a photocurable resin. To prevent polymerizing independent of the active species generated from a photopolymerizable initiator, substances soluble an aqueous solvent at 80°C or lower are especially preferably used. Specific examples include itaconic acid, *N,N'*-methylenebisacrylate, hydroxyethyl methacrylate, hydroxypropyl methacrylate, *N,N'*-methylenebisacrylamide, *N*-isopropylacrylamide, *N*-vinylpyrrohdone, acryloylmorpholine, *N,N'*-dimethylacrylamide, *N*-vinylfomlamide, (meth)acrylates, and the like. Water soluble compounds having an unsaturated bond may be used alone or in a combination of two or more. The water soluble compound having an unsaturated bond is contained in the shell composition in a solid content of 0.01 to 30 wt% and preferably 0.1 to 25 wt%.

A polymerization initiator is not particularly limited, and a photopolymerization initiator is preferably used. The photopolymerization initiator is a compound that generates a polymerization initiating species and accerlates polymerization or cross-linking with photoirradiation. Typical examples thereof include benzoin, acetoin, benzoin methyl ether, benzoin ethyl ether, benzoin isopropyl ether, benzophenone, benzyl, Michler's ketone, xanthone, chlorothioxanthone, isopropylthioxanthon, benzyl dimethyl ketal, naphthol, anthraquinone, hydroxyanthracene, acetophenone diethyl ketal, α-hydroxycyclohexyl phenyl ketone, 2-hydroxy-2-methylphenylpropane, aromatic iodonium salts, aromatic sulfonium salts, iodonium salts, sulfonium salts, triarylsulfonium salts, trifluorocarbon sulfonium salts, and the like. Polymerization initiators may be used alone or in a combination of two or more. The polymerization initiator is contained in the shell composition in a solid content of 0.001 to 20 wt% and preferably 0.1 to 10 wt%.

A photosensitizer is contained, with a photocurable resin as a water permeable substance. Examples of photosensitizers include ruthenium complexes, porphyrin-based compounds, and the like. Such a photosensitizer gives sufficient sensitivity to the visible light region.

A coloring agent is not particularly limited, and natural coloring agents, artificial coloring agents, and the like are suitably used as necessary.

Examples of pore-forming agents include starch, modified starch (such as alkylated starch and etherified starch), and macromolecules, such as dextrin, cellulose, and protein, having an average molecular weight of 1000 or greater. A pore-forming agent is used to further enhance the permeability of shell for a resulting seamless capsule. For example, the capsule is formed with a pore-forming agent, and the pore-forming agent is then removed from the shell of the capsule by enzymatic treatment, acid or alkali treatment, or the like (for example, for cleavage, degradation, dissolution of macromolecule), so that pores can be formed or the diameter of the pores can be enlarged in the shell of the capsule, resulting in capsules with high permeability of shell.

### (3) Preparation of two-layered seamless capsule

The two-layered seamless capsule (Fig. 1 (a)) is prepared by, for example, a method of dropping in fluid using a capsule production device equipped with a concentric double nozzle, or a like method. Specifically, the capsule production device equipped with a concentric double nozzle having, from the innermost, a first nozzle and a second nozzle is used and the method includes a step of simultaneously extruding a suspension composition that contains a biocatalyst suspended in an oily substance from the first nozzle and a shell composition that contains a water permeable substance from the second nozzle into a fluid. For this fluid, a liquid oily substance (liquid oil) such as a foregoing oily substance that is liquid at 0 to 40°C may be used. This fluid is preferably used with cooling.

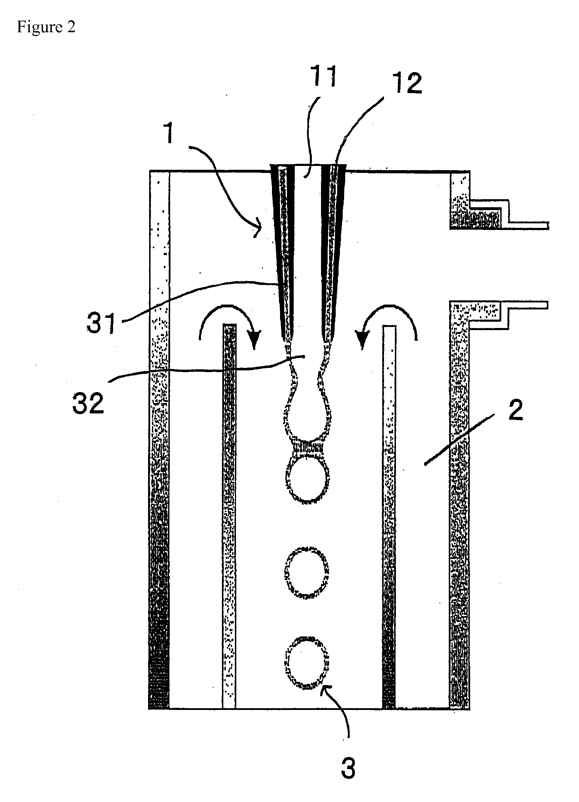

The two-layered seamless capsule is produced by, for example, a method of dropping in fluid using a device for production as shown in Fig. 2. In the device, a concentric double nozzle 1 is disposed in a carrier fluid 2 flowing downward at constant rate, with the outlet of the nozzle faces downward. When contents 32 to be encapsulated (a suspension composition that contains a biocatalyst suspended in an oily substance) and an outer layer composition (a shell composition) 31 are simultaneously extruded (injected) at a constant rate into a fluid from the innermost nozzle (an inner nozzle 11) and the outer nozzle (an outer nozzle 12) of the concentric double nozzle 1, respectively, the surface tension that acts between the carrier fluid 2 and the outer layer composition 31 forms a jet having a two-layer structure, which then turns into spherical droplets. Vibrations may be applied to the jet stream to enhance uniformity in the size of the resulting seamless capsules.

Then, the droplets are cooled in the carrier fluid 2, or the droplets are photoirradiated in the carrier fluid 2 or after isolated from the carrier fluid 2 to form a shell on the surface of the droplets. Where the shell composition contains a gelling agent, the shell is formed by sol-gel transition with cooling. Where the shell composition contains a photocurable resin, the shell is formed by photoirradiation. In use of alginate, gellan gum, pectin, or carrageenan as a gelling agent, the dropping may be carried out in a solution of a divalent or higher valent metal ion. Thus, a two-layered seamless capsule 3 is obtained.

Where the shell composition contains a gelling agent, the temperature for solidifying is set to be in the range of -10 to 30°C and preferably 0 to 20°C.

Where the shell composition contains a photocurable resin, the wavelength of activating light necessary for curing the photocurable resin is generally in the range of about 200 nm to about 600 nm, and it is advantageous to perform irradiation using a light source that emits light having such a wavelength. Such light sources include mercury lamps, fluorescent lamps, xenon lamps, carbon arc lamps, metal halide lamps, and the like. It is preferable that the foregoing shell composition further contains a photosensitizer to give sufficient sensitivity to the visible light region. Depending on the type of photocurable resin, active ray having a short wavelength in the ultraviolet region can also be used for curing. Although the time for irradiation varies depending on the intensity of and the distance from a light source, it is generally in the range of 0.05 seconds to 10 minutes and preferably 0.5 seconds to 2 minutes.

The diameter range of the two-layered seamless capsule is usually 0.1 mm to 10 mm and preferably 0.3 mm to 8 mm. Depending on the application, the prepared capsule may be used while water remaining in the capsule without drying or may be dried by drying under atmospheric pressure or vacuum drying before use.

The two-layered seamless capsule has no seams and are uniform in size and thickness of shell. A variety of additives such as a coloring agent can be used in the shell composition of the two-layered seamless capsule to prepare a capsule having the desired features as with conventional capsules.

Seamless capsule of the present invention

The seamless capsule of the present invention is obtained by immersing the two-layered seamless capsule as described above in an aqueous fluid. For example, water or an aqueous solution containing an aqueous component such as a liquid medium as mentioned above is used for the aqueous fluid. The amount of aqueous fluid is not particularly limited as long as it allows the seamless capsule to be immersed. The conditions for immersion also are not particularly limited. To prevent the inactivation of the biocatalyst contained in the seamless capsule, the immersion is performed, for example, at 4 to 60°C for 1 hour to 60 days. The immersion is preferably performed with stirring in that the aqueous fluid can be efficiently encapsulated.

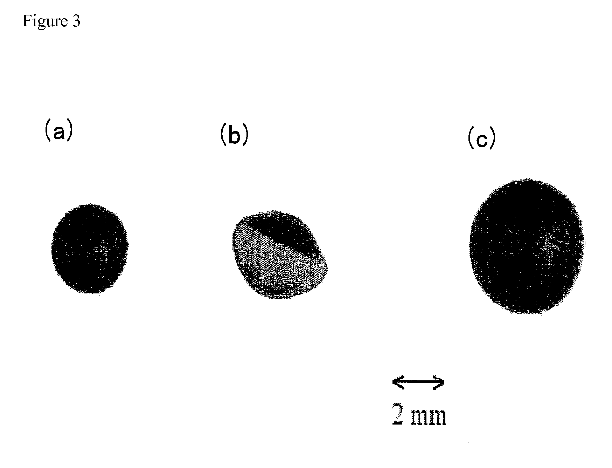

As for an example of production of the seamless capsule of the present invention, Fig. 3 shows the state change of the two-layered seamless capsule during immersing the capsule in an aqueous fluid. When the dry two-layered seamless capsule as shown in Fig. 3(a) is immersed in, for example, water at ordinary temperatures (about 20°C) for about 20 minutes, a depressed spherical capsule as shown in Fig. 3(b) is formed, which shows that the outer layer (shell) partially extends so that the aqueous fluid is incorporated into the capsule and a layer of the aqueous fluid is formed in the capsule. Subsequently, the immersion is continued (for example, 6-hour immersion), thereby the shell of the outer layer further expands to gradually reduce the depression of the capsule, leading to a spherical seamless capsule as shown in Fig. 3(c). The seamless capsule is prepared using, for example, the oily substance of the inner layer which has been previously colored with a coloring agent or the like, and a colored layer in form of sphere as shown in Fig. 1(b) is formed in the spherical seamless capsule. Therefore, it is clear that a pseudo three-layer structure of outer layer/oil layer/aqueous layer is formed in the seamless capsule of the present invention.

Regarding the seamless capsule of the present invention, a biocatalyst and an aqueous fluid are brought into contact, and the biocatalyst is activated to produce a useful substance. For example, a two-layered seamless capsule having a biocatalyst such as a microorganism encapsulated is immersed in a liquid medium to incorporate the liquid medium thereinto, thereby giving the seamless capsule of the present invention. In this seamless capsule, the microorganism grows and produces a metabolite (useful component) by utilizing components of the medium. This metabolite then passes through the outer layer to be released from the inside to the outside of the capsule. Accordingly, the seamless capsule of the present invention can be used as a bioreactor.

Also, the seamless capsule of the present invention can be used as a sustained-release drug. For example, the seamless capsule having islets of Langerhans encapsulated can be administered to a patient who has developed diabetes to make the blood-sugar level to be maintained within a normal range for a long period of time.

### Examples

### Reference Example 1: Investigation for shell (outer layer) permeability 1

A mixture of 60 parts by weight of a 40% ENTG-3800 (manufactured by Kansai Paint Co., Ltd.) in aqueous solution, 0.6 parts by weight of acetoin, and 20 parts by weight of a 0.5% poval (an average molecular weight of about 9000) in aqueous solution was prepared as a shell composition. A mixture of 100 parts by weight of soybean oil and 20 parts by weight of liquid paraffin was prepared as an oily substance, and 20 parts by weight of dextrin having an average molecular weight of about 1000 was suspended in the oily substance to prepare a suspension composition.

A capsule production device equipped with a concentric double nozzle as shown in Fig. 2 was provided with circulating vegetable oil with cooling to 15°C as a carrier fluid. The shell composition (outer layer composition) and the suspension composition were injected from the outer nozzle and from the inner nozzle, respectively, of this device, into the carrier fluid, such that the two-layer combined jet was formed at a specific rate (540 mm/second), thereby giving droplets (seamless capsules).

The droplets (seamless capsules) in the carrier fluid were irradiated with ultraviolet rays using a high pressure mercury lamp (wavelength of 320 to 400 nm) to polymerize the photocurable resin (ENTG-3800). The diameter of the resulting seamless capsules was 4 mm.

Twenty of the seamless capsules thus obtained were collected in a 50 ml beaker. Next, 30 ml of distilled water was added to the beaker to immerse the seamless capsules therein and stirred at 20°C for 20 hours, and the dextrin concentration in water was measured over time using the phenol-sulfuric acid method. Table 1 shows the results. There were no broken seamless capsules when stirring was terminated.

**Table 1**

| Time (h) | Dextrin concentration (in terms of glucose) (mM) |
|---|---|
| 0 | 0 |
| 5 | 1.9 |
| 10 | 7.5 |
| 20 | 10.9 |

As seen in the results presented in Table 1, the dextrin concentration in water was increased over time, which shows that the dextrin having an average molecular weight of about 1000 in the seamless capsule was dissolved in water that had penetrated through the shell (outer layer) from the outside of the capsules and entered into the capsules, and then was released from the inside of the capsules through the shell to the outside.

### Reference Example 2: Investigation for shell (outer layer) permeability 2

Seamless capsules having a diameter of 4 mm were obtained in the same manner as in Example 1, except that povidone (having an average molecular weight of about 1,300,000) was used in place of poval to prepare a shell composition and pullulan having an average molecular weight of about 120,000 was used in place of dextrin having an average molecular weight of about 1,000 to prepare a suspension composition. Also, the pullulan concentration was measured over time in the same manner as in Example 1. Table 2 shows the results. There were no broken seamless capsules when stirring was terminated.

**Table 2**

| Time (h) | Pullulan concentration (in terms of glucose) (mM) |
|---|---|
| 0 | 0 |
| 5 | 1.1 |
| 10 | 3.9 |
| 20 | 6.7 |

As seen in the results presented in Table 2, the pullulan concentration in water was increased over time, which shows that the pullulan having an average molecular weight of about 120,000 in the seamless capsules was dissolved in water that had penetrated through the shell (outer layer) from the outside of the capsules and entered into the capsules, and then was released from the inside of the capsules through the shell to the outside. Even such a macromolecule can pass the outer layer.

### Reference Example 3: Investigation for shell (outer layer) permeability 3

A mixture of 80 parts by weight of a 40% ENT-3400 (manufactured by Kansai Paint Co., Ltd.) in aqueous solution and 0.6 parts by weight of benzoin isobutyl ether was prepared as a shell composition. A mixture of 100 parts by weight of soybean oil and 20 parts by weight of liquid paraffin was prepared as an oily substance, and 20 parts by weight of dextrin having an average molecular weight of about 1000 was suspended in the oily substance to prepare a suspension composition.

The shell composition and the suspension composition were injected using a capsule production device equipped with a concentric double nozzle in the same manner as in Reference Example 1, thereby giving droplets (seamless capsules).

The droplets (seamless capsules) were irradiated with ultraviolet rays using a high pressure mercury lamp (wavelength of 320 to 400 nm) to polymerize the photocurable resin (ENT-3400). The diameter of the resulting seamless capsules was 4 mm. The seamless capsules were then dried to give dry seamless capsules having a diameter of 3.5 mm.

Twenty of the dry seamless capsules thus obtained were collected in a 100 ml beaker. Next, 50 ml of distilled water was added to the beaker to immerse the seamless capsules therein and the capsules were left to stand still at 20°C for 6 hours. Ten of the immersed seamless capsules were removed, the thickness of the shell and the diameter of the capsules were measured, and their averages were determined. Moreover, the volume of the shell was calculated based on the measured values of the thickness of the shell and the diameter of the capsules. Table 3 shows the results.

**Table 3**

| | Thickness of shell (µm) | Capsule size (mm) | Volume of shell (mm³) |
|---|---|---|---|
| Before immersion in water | 144 | 3.5 | 5.1 |
| After immersion in water | 152 | 5.0 | 7.0 |

As seen in the results presented in Table 3, the seamless capsules after immersion in water had a much greater diameter while not differing greatly in the thickness or the volume of the shell, compared to the seamless capsules before immersion in water, which shows that by the immersion of the seamless capsule in water, the shell itself was not hardly swelled with water and water was transferred to the inside of the seamless capsules.

### Example 1

Lactic acid bacterium (*Lactococcus lactis* subsp. *lactis* JCM 7638) was subjected to stationary culture in deMann-Rogosa-Sharpe (MRS) broth medium (manufactured by Oxoid Limited) with marble at 37°C for 15 hours. The viable cell count in the resulting culture suspension was 1.5×10¹⁰ cfu/ml. The "cfu" is an abbreviation for colony forming unit and denotes a viable cell count. The culture suspension was centrifuged under the condition of 10000 x g at 4°C for 20 minutes, and the resulting precipitate was freeze-dried to prepare lactic acid bacterial powder.

Twenty parts by weight of PEG 1000 (polyethylene glycol having an average molecular weight of about 1000) was added to 180 parts by weight of a 2% agar in aqueous solution maintained at 80°C to prepare a shell composition. The lactic acid bacterial powder was suspended in a mixture of 85 parts by weight of a long chain triglyceride and 15 parts by weight of a sucrose fatty acid ester to prepare a suspension composition.

A capsule production device equipped with a concentric double nozzle as shown in Fig. 2 was provided with circulating vegetable oil with cooling to 9°C as a carrier fluid. The shell composition (outer layer composition) and the suspension composition were injected from the outer nozzle and from the inner nozzle, respectively, of this device, into the carrier fluid, such that the two-layer combined jet was formed at a specific rate (555 mm/second), thereby giving two-layered seamless capsules having a diameter of 4 mm.

Twenty parts by weight of the two-layered seamless capsules, after isolated from the carrier fluid (vegetable oil) and washed with sterile physiological saline, were subjected to shaking culture under the condition of 100 cpm at 37°C using 100 parts by weight of liquid MRS medium (pH 6.5, glucose concentration of 0.3 M), thereby giving seamless capsules having a pseudo three-layer structure of outer layer/oil layer/aqueous layer. The lactic acid concentration and the glucose concentration in the liquid MRS medium were measured over time. The lactic acid concentration was measured using Lactate assay kit (manufactured by Funakoshi Corporation), and the glucose concentration was measured using Somogyi-Nelson method. Table 4 shows the results. The culturing was carried out for 48 hours in all.

**Table 4**

| Time (h) | Glucose concentration (M) | Lactic acid concentration (M) |
|---|---|---|
| 0 | 0.30 | 0 |
| 12 | 0.15 | 0.13 |
| 24 | 0.09 | 0.20 |
| 36 | 0.01 | 0.28 |
| 48 | 0 | 0.29 |

As seen in the results presented in Table 4, during the process of the culturing, the glucose concentration was decreased and the lactic acid concentration was increased in the liquid MRS medium.

After culturing, 10 of the cultured seamless capsules were collected and washed with water to remove the medium. Each seamless capsule was crushed and the viable cell count in the contents of the seamless capsules was determined using MRS agar medium (manufactured by Oxoid Limited) to be 2.3×10¹⁰ cfu/capsule, which was greater than that before culturing (8.1×10⁸ cfu/capsule). Moreover, the diameter of the resulting seamless capsules was increased from 4 mm to 4.5 mm.

Meanwhile, the viable cell count and the pH in the liquid MRS medium used for the shaking culture were determined. The results showed that the viable cell count was below the detection limit and the pH was 4.0, which was lower than pH 6.5 before culturing. It could be understood from the result of viable cell count that the lactic acid bacterium did not escape from the seamless capsules. Moreover, given the result of pH and the increase over time in lactic acid concentration as presented in Table 4, it seems that lactic acid produced by the lactic acid bacterium was released through the shell of the seamless capsules to the outside of the capsules.

It was shown from the results provided above that the seamless capsule of the present invention is useful as a bioreactor.

### Example 2

A mixture of 60 parts by weight of a 40% ENT-3400 (manufactured by Kansai Paint Co., Ltd.) in aqueous solution, 0.6 parts by weight of benzoin isobutyl ether, and 20 parts by weight of a 1% acryloylmorpholine (manufactured by Kohjin Co., Ltd.) in aqueous solution was prepared as a shell composition. A mixture of 100 parts by weight of soybean oil and 20 parts by weight of liquid paraffin was prepared as an oily substance, and 20 parts by weight of compressed baker's yeast (manufactured by Oriental Yeast Co., Ltd.) was suspended in the oily substance to prepare a suspension composition. The shell composition and the suspension composition were injected using a capsule production device equipped with a concentric double nozzle in the same manner as in Reference Example 1, thereby giving droplets (two-layered seamless capsules).

The droplets (two-layered seamless capsules) were irradiated with ultraviolet rays using a high pressure mercury lamp (wavelength of 320 to 400 nm) to polymerize the photocurable resin (ENT-3400). The diameter of the resulting seamless capsules was 4 mm.

Twenty parts by weight of the two-layered seamless capsules, after isolated from the carrier fluid (vegetable oil) and washed with sterile physiological saline, were subjected to shaking culture under the condition of 100 cpm at 37°C for 48 hours using 100 parts by weight of liquid YM medium (prepared by dissolving 10 g of glucose, 2 g of yeast extract, 2 g of malt extract, and 2 g of soy peptone in 11 of distilled water, the pH adjusted to 6.2), thereby giving seamless capsules having a pseudo three-layer structure of outer layer/oil layer/aqueous layer. It was observed that carbon dioxide was generated on the surface of the shell during the culturing.

After culturing, 10 of the seamless capsules were collected and washed with water to remove the medium. Each seamless capsule was crushed and the viable cell count in the contents of the seamless capsules was determined using YM agar medium (prepared by suspending 10 g of glucose, 2 g of yeast extract, 2 g of malt extract, 2 g of soy peptone, and 20 g of agar in 11 of distilled water, the pH adjusted to 6.2, heated at 121°C for 15 minutes and cooled to solidify). The result was 9.3×10⁸ cfu/capsule, which was greater than that before culturing (8.1×10⁷ cfu/capsule).

Meanwhile, the viable cell count in the liquid YM medium used for shaking culture was determined. The result showed that the viable cell count was below the detection limit.

Moreover, after the seamless capsules cultured in the liquid YM medium were washed with water to remove the medium, 50 parts by weight of the seamless capsules were subjected to shaking culture under the condition of 20 cpm at 30°C using the liquid glucose medium (prepared by dissolving 54 g of glucose in 0.1 mol/l of phosphate buffer (pH 6.0)). The ethanol concentration and the glucose concentration in the liquid glucose medium were measured over time. The ethanol concentration was measured by gas chromatography performed under the conditions specified below The glucose concentration was measured using Somogyi-Nelson method. Table 5 shows the results. The culturing was carried out for 48 hours in all.

Gas chromatography conditions
Internal standard: Acetone
Column: Glass column having an inner diameter of 3 mm and a length of 2 m
Stationary phase: Polyethylene glycol 1000 (10%, passed through 60-80 mesh)
Injection temperature: 200°C
Column temperature: 100°C
Detector: Hydrogen flame ionization detector
Detector temperature: 150°C
Carrier gas: Nitrogen
Flow rate: 30 to 40 ml/min

**Table 5**

| Time (h) | Glucose concentration (M) | Ethanol concentration (M) |
|---|---|---|
| 0 | 0.30 | 0 |
| 12 | 0.24 | 0.03 |
| 24 | 0.15 | 0.07 |
| 36 | 0.10 | 0.11 |
| 48 | 0.04 | 0.13 |

As seen in the results presented in Table 5, during the process of the culturing, the glucose concentration was decreased and the ethanol concentration was increased in the liquid glucose medium. It could be understood that glucose was consumed and ethanol was produced by the yeast.

### Example 3

A mixture of 60 parts by weight of a 40% nona(ethyleneglycol)diacrylate in aqueous solution, 0.6 parts by weight ofbenzoin isobutyl ether, and 20 parts by weight of a 30% calcium acrylate in aqueous solution was prepared as a shell composition. Separately, mouse islet of Langerhans cells were cultured to 80% confluent on Dulbecco's modified Eagle's medium (DMEM) (manufactured by Difco), treated with trypsin, and centrifuged under the condition of 5000 x g at 4°C. A mixture of 60 parts by weight of soybean oil and 60 parts by weight of liquid paraffin was prepared as an oily substance, and the resulting islet of Langerhans cells were suspended in the oily substance to be 5×10³ cells/ml to prepare a suspension composition. The shell composition and the suspension composition were injected using a capsule production device equipped with a concentric double nozzle in the same manner as in Reference Example 1, thereby giving droplets (two-layered seamless capsules).

The droplets (two-layered seamless capsules) were irradiated with ultraviolet rays using a high pressure mercury lamp (wavelength of 320 to 400 nm) to polymerize the photocurable resin (nona(ethyleneglycol)diacrylate). The diameter of the resulting seamless capsules was 4 mm.

Streptozotocin was intraperitoneally administered to a DDY mouse having a body weight of 20 g (male, 6 weeks old) in a dose of 200 mg/kg. Then, the blood glucose level of the mouse was measured to be 422 mg/dl, and it was confirmed that the mouse developed diabetes (blood glucose level of 300 mg/dl or greater).

Next, three days after the blood glucose level was measured, the seamless capsule having islets of Langerhans encapsulated as described above was implanted into the diabetic mouse intraperitoneally The blood glucose level was measured at given times. Table 6 shows the results.

**Table 6**

| Days after implantation of seamless capsule (Day) | Blood glucose level (mg/dL) |
|---|---|
| 0 | 460 |
| 3 | 150 |
| 6 | 88 |
| 12 | 102 |
| 18 | 132 |
| 27 | 145 |
| 36 | 200 |

The results presented in Table 6 show that after the implantation of the seamless capsule into the diabetic mouse, the blood glucose levels were observed to be at normal levels over a long period of time, exhibiting therapeutic effects on diabetes. The implanted seamless capsule was removed, and the removed capsule had a pseudo three-layer structure of outer layer/oil layer/aqueous layer. Note that there was no rejection to the seamless capsule observed during the test period.

### Comparative Example 1

A mixture of 60 parts by weight of a 40% ENTG-3800 (manufactured by Kansai Paint Co., Ltd.) in aqueous solution, 0.6 parts by weight of benzoin isobutyl ether, 20 parts by weight of a 1% acryloylmorpholine (manufactured by Kohjin Co., Ltd.) in aqueous solution, and 20 parts by weight of freeze-dried lactic acid bacterial powder as obtained in Example 1 was prepared as a shell composition.

A capsule production device equipped with a concentric double nozzle as shown in Fig. 2 was provided with circulating vegetable oil with cooling to 9°C as a carrier fluid. The shell composition (outer layer composition) was injected from the inner nozzle of the device into the carrier fluid such that the jet was formed at a specific rate (555 mm/second), thereby giving beads having a diameter of 4 mm.

The beads were isolated from the carrier fluid (vegetable oil) and washed with sterile physiological saline. Ten of the resulting beads were crushed, and the viable cell count was determined using MRS agar medium to be 3.5×10⁹ cfu/bead. Twenty parts by weight of the beads were placed in 100 parts by weight of liquid MRS medium (pH 6.5, glucose concentration of 0.3 M) and subjected to shaking culture under the condition of 100 cpm at 37°C for 48 hours.

After culturing, 10 of the cultured beads were collected and washed with water to remove the medium. Each bead was crushed and the viable cell count in the beads was determined using MRS agar medium. As a result, the viable cell count was 9.1×10⁹ cfu/bead and was greater than that before culturing (3.5x 10⁹ cfu/bead). Also, the viable cell count in the medium was determined to be 5.5×10⁷ cfu/ml. These results show that lactic acid bacterium escaped out of the beads.

As can be clearly understood from the results of the Examples and the Comparative Example above, the seamless capsule of the present invention is useful as a bioreactor.

### Industrial Applicability

According to the invention, a seamless capsule having a pseudo three-layer structure of outer layer/oil layer/aqueous layer is obtained by immersing in an aqueous fluid a two-layered seamless capsule composed of an inner layer of a suspension composition that contains a biocatalyst such as an enzyme and a living cell suspended in an oily substance and an outer layer of a shell composition that contains a water permeable substance. In the two-layered seamless capsule, the biocatalyst is suspended in the oily substance in the inner layer, and is stable for a long period of time. Also, the outer layer that allows an aqueous fluid to penetrate allows only an aqueous liquid to transfer between the inside and the outside of the capsule without allowing the biocatalyst to pass to the outside. Therefore, when the two-layered seamless capsule is immersed in an aqueous fluid, the aqueous fluid is incorporated into the inside (inner layer) of the capsule and a layer of the aqueous fluid is formed, thereby giving a pseudo three-layer structure of outer layer/oil layer/aqueous layer. Then, the biocatalyst in the capsule is activated by the incorporated aqueous fluid to produce a useful substance. For example, by immersing in a liquid medium a seamless capsule having a microorganism (biocatalyst) encapsulated, the liquid medium can be incorporated into the capsule while immobilizing the microorganism in the capsule and a metabolite (useful substance) produced by the microorganism can be released from the inside to the outside of the capsule. Thus, the seamless capsule of the present invention is useful as a bioreactor.

### Description of the Numerals

1 Concentric double nozzle
2 Carrier fluid
3 Seamless capsule
11 Inner nozzle
12 Outer nozzle
31 Outer layer composition
32 Contents

## Claims

1. A seamless capsule that can be stably stored for a long period of time in the form of a two-layered seamless capsule, comprising
- an inner layer comprising a suspension composition, and
- an outer layer comprising a shell composition and having water permeability,
wherein the suspension composition of the inner layer comprises a biocatalyst suspended in an oily substance, and the shell composition of the outer layer comprises a water permeable substance,
wherein the two-layered seamless capsule is forming a pseudo three-layer structure of outer layer/oil layer/aqueous layer, when being immersed in an aqueous fluid; wherein the biocatalyst is at least one selected from the group consisting of microorganisms, animal cells, plant cells, and plant tissues.

2. The seamless capsule according to claim 1, wherein the biocatalyst is at least one selected from the group consisting of microorganisms, animal cells, plant cells, and plant tissues, and the biocatalyst is activated by the pseudo three-layer structure of outer layer/oil layer/aqueous layer.

3. The seamless capsule according to any one of claims 1 or 2, wherein the shell composition comprises at least one selected from the group consisting of carrageenan, agar, glucomannan, alginate, acrylate-based oligomers, unsaturated polyester-based oligomers, epoxy-based oligomers, vinyl ether-based oligomers, polyene-thiol-based oligomers, and cinnamate-based oligomers.

4. The seamless capsule according to any one of claims 1 to 3, wherein the oily substance is at least one selected from the group consisting of olive oil, jojoba oil, corn oil, rapeseed oil, lard, beef tallow, whale oil, castor oil, soybean oil, rice oil, rice germ oil, coconut oil, palm oil, cacao seed oil, avocado oil, macadamia nut oil, squalane, mink oil, turtle oil, hydrocarbons having 8 to 30 carbon atoms, beeswax, carnauba wax, rice wax, lanolin, liquid paraffin, petrolatum, fatty acids having 4 to 30 carbon atoms, esters of fatty acids having 4 to 30 carbon atoms and sucrose, esters of fatty acids having 4 to 30 carbon atoms and glycerol, fatty alcohols having 4 to 30 carbon atoms, and esters of fatty acids having 4 to 30 carbon atoms and fatty alcohols having 4 to 30 carbon atoms.

5. A bioreactor comprising the seamless capsule of any one of claims 1 to 4.

6. A method for producing the seamless capsule according to any one of claims 1 to 4, comprising immersing in an aqueous fluid a two-layered seamless capsule, comprising
- an inner layer comprising a suspension composition, and
- an outer layer comprising a shell composition and having water permeability,
wherein the suspension composition of the inner layer comprises a biocatalyst suspended in an oily substance and the shell composition of the outer layer comprises a water permeable substance.

7. The method according to claim 6, wherein the two-layered seamless capsule is obtained by using a capsule production device equipped with a concentric double nozzle having, from the innermost, a first nozzle and a second nozzle, by a method comprising simultaneously extruding the suspension composition comprising a biocatalyst suspended in an oily substance from the first nozzle and the shell composition comprising a water permeable substance from the second nozzle into a fluid.

8. Use of the seamless capsule according to any one of claims 1 to 4 as a bioreactor.

## Patentansprüche

1. Nahtlose Kapsel, die stabil für einen langen Zeitraum gelagert werden kann, in Form einer zweischichtigen nahtlosen Kapsel, umfassend:
- eine innere Schicht, umfassend eine Suspensions-Zusammensetzung; und
- eine äußere Schicht, umfassend eine Hüllen-Zusammensetzung und aufweisend Durchlässigkeit für Wasser;
wobei die Suspensions-Zusammensetzung der inneren Schicht einen in einer öligen Substanz suspendierten Biokatalysator umfasst und die Hüllen-Zusammensetzung der äußeren Schicht eine für Wasser durchlässige Substanz umfasst;
wobei die zweischichtige nahtlose Kapsel eine Pseudo-Dreischichten-Struktur aus äußerer Schicht / Öl-Schicht / wässriger Schicht bildet, wenn sie in ein wässriges Fluid eingetaucht wird, wobei der Biokatalysator wenigstens einer ist, der gewählt ist aus der Gruppe, die besteht aus Mikroorganismen, Tier-Zellen, Pflanzen-Zellen und Pflanzen-Geweben.

2. Nahtlose Kapsel nach Anspruch 1, worin der Biokatalysator wenigstens einer ist, der gewählt ist aus der Gruppe, die besteht aus Mikroorganismen, Tier-Zellen, Pflanzen-Zellen und Pflanzen-Geweben, und der Biokatalysator aktiviert wird durch die Pseudo-Dreischichten-Struktur aus äußerer Schicht / Öl-Schicht / wässriger Schicht.

3. Nahtlose Kapsel nach irgendeinem der Ansprüche 1 oder 2, wobei die Hüllen-Zusammensetzung wenigstens eine Verbindung umfasst, die gewählt ist aus der Gruppe, die besteht aus Carrageen, Agar, Glucomannan, Alginat, Acrylat-basierten Oligomeren, ungesättigten Polyesterbasierten Oligomeren, Epoxy-basierten Oligomeren, Vinylether-basierten Oligomeren, Polyenthiol-basierten Oligomeren und Cinnamat-basierten Oligomeren.

4. Nahtlose Kapsel nach irgendeinem der Ansprüche 1 bis 3, wobei die ölige Substanz wenigstens eine ist, die gewählt ist aus der Gruppe, die besteht aus Olivenöl, Jojoba-Öl, Maisöl, Rapssamen-Öl, Schmalz, Rindertalg, Walöl, Rhizinusöl, Sojabohnen-Öl, Reisöl, Reiskeim-Öl, Kokosnuss-Öl, Palmöl, Kakaosamen-Öl, Avocado-Öl, Macadamianuss-Öl, Squalan, Nerzöl, Schildkröten-Öl, Kohlenwasserstoffen mit 8 bis 30 Kohlenstoff-Atomen, Bienenwachs, Carnaubawachs, Reiswachs, Lanolin, flüssiges Paraffin, Petrolatum, Fettsäuren mit 4 bis 30 Kohlenstoff-Atomen, Estern von Fettsäuren mit 4 bis 30 Kohlenstoff-Atomen und Rohrzucker, Estern von Fettsäuren mit 4 bis 30 Kohlenstoff-Atomen und Glycerin, Fettalkoholen mit 4 bis 30 Kohlenstoff-Atomen und Estern von Fettsäuren mit 4 bis 30 Kohlenstoff-Atomen und Fettalkoholen mit 4 bis 30 Kohlenstoff-Atomen.

5. Bioreaktor, umfassend die nahtlose Kapsel nach irgendeinem der Ansprüche 1 bis 4.

6. Verfahren zum Herstellen der nahtlosen Kapsel nach irgendeinem der Ansprüche 1 bis 4, umfassend ein Eintauchen einer zweischichtigen nahtlosen Kapsel in ein wässriges Fluid, umfassend
- eine innere Schicht, umfassend eine Suspensions-Zusammensetzung; und
- eine äußere Schicht, umfassend eine Hüllen-Zusammensetzung und aufweisend Durchlässigkeit für Wasser;
- wobei die Suspensions-Zusammensetzung der inneren Schicht einen in einer öligen Substanz suspendierten Biokatalysator umfasst und die Hüllen-Zusammensetzung der äußeren Schicht eine für Wasser durchlässige Substanz umfasst;

7. Verfahren nach Anspruch 6, wobei die zweischichtige nahtlose Kapsel erhalten wird unter Verwendung einer Kapsel-Produktionsvorrichtung, die ausgestattet ist mit einer konzentrischen Doppel-Düse, die aufweist - von ihrer innersten Düse - eine erste Düse und eine zweite Düse, mittels eines Verfahrens, das umfasst ein gleichzeitiges Extrudieren der Suspensions-Zusammensetzung, die einen Biokatalysator suspendiert in einer öligen Substanz umfasst, von der ersten Düse und der Hüllen-Zusammensetzung, die eine für Wasser durchlässige Substanz umfasst, von der zweiten Düse in ein Fluid.

8. Verwendung der nahtlosen Kapsel nach irgendeinem der Ansprüche 1 bis 4 als Bioreaktor.

## Revendications

1. Capsule sans soudure qui peut être conservée de manière stable durant une longue période de temps sous la forme d'une capsule sans soudure à deux couches, comprenant
- une couche interne comprenant une composition de suspension et
- une couche externe comprenant une composition de coque et ayant une perméabilité à l'eau,
dans laquelle la composition de suspension de la couche interne comprend un biocatalyseur en suspension dans une substance huileuse, et la composition de coque de la couche externe comprend une substance perméable à l'eau,
dans laquelle la capsule sans soudure à deux couches forme une pseudo structure à trois couches de couche externe / couche huileuse / couche aqueuse, quand elle est immergée dans un liquide aqueux ; dans laquelle le biocatalyseur est au moins un choisi dans le groupe constitué par les microorganismes, les cellules animales, les cellules végétales et les tissus végétaux.

2. Capsule sans soudure selon la revendication 1, dans laquelle le biocatalyseur est au moins un choisi dans le groupe constitué par les microorganismes, les cellules animales, les cellules végétales et les tissus végétaux, et le biocatalyseur est activé par la pseudo structure à trois couches de couche externe / couche huileuse / couche aqueuse.

3. Capsule sans soudure selon l'une quelconque des revendications 1 ou 2, dans laquelle la composition de coque comprend au moins un choisi dans le groupe constitué par le carraghénane, l'agar, le glucomannane, l'alginate, les oligomères à base d'acrylate, les oligomères à base de polyester insaturé, les oligomères à base d'époxy, les oligomères à base d'éther vinylique, les oligomères à base de polyène-thiol et les oligomères à base de cinnamate.

4. Capsule sans soudure selon l'une quelconque des revendications 1 à 3, dans laquelle la substance huileuse est au moins une choisie dans le groupe constitué par l'huile d'olive, l'huile de jujube, l'huile de maïs, l'huile de graine de colza, le lard, le suif de boeuf, l'huile de baleine, l'huile de ricin, l'huile de soja, l'huile de riz, l'huile de germe de riz, l'huile de noix de coco, l'huile de palme, l'huile de graine de cacao, l'huile d'avocat, l'huile de macadamia, le squalane, l'huile de vison, l'huile de tortue, les hydrocarbures ayant 8 à 30 atomes de carbone, la cire d'abeilles, la cire de carnauba, la cire de riz, la lanoline, la paraffine liquide, le pétrolatum, les acides gras ayant 4 à 30 atomes de carbone, les esters d'acides gras ayant 4 à 30 atomes de carbone et le saccharose, les esters d'acides gras ayant 4 à 30 atomes de carbone et le glycérol, les alcools gras ayant 4 à 30 atomes de carbone, et les esters d'acides gras ayant 4 à 30 atomes de carbone et les alcools gras ayant 4 à 30 atomes de carbone.

5. Bioréacteur comprenant la capsule sans soudure selon l'une quelconque des revendications 1 à 4.

6. Procédé de production de la capsule sans soudure selon l'une quelconque des revendications 1 à 4, comprenant l'immersion dans un liquide aqueux d'une capsule sans soudure à deux couches, comprenant
- une couche interne comprenant une composition de suspension et
- une couche externe comprenant une composition de coque et ayant une perméabilité à l'eau,
dans lequel la composition de suspension de la couche interne comprend un biocatalyseur en suspension dans une substance huileuse et la composition de coque de la couche externe comprend une substance perméable à l'eau.

7. Procédé selon la revendication 6, dans lequel la capsule sans soudure à deux couches est obtenue en utilisant un dispositif de production de capsule équipé d'une double buse concentrique ayant, depuis la partie la plus interne, une première buse et une seconde buse, par un procédé comprenant l'extrusion simultanée de la composition de suspension comprenant un biocatalyseur en suspension dans une substance huileuse de la première buse et la composition de coque comprenant une substance perméable dans l'eau de la seconde buse dans un liquide.

8. Utilisation de la capsule sans soudure selon l'une quelconque des revendications 1 à 4 comme bioréacteur.
